# FASCICULE DE BREVET EUROPEEN

(11) **EP 1 362 845 B1**
(45) Date de publication et mention de la délivrance du brevet: **14.10.2009**
(21) Numéro de dépôt: 03292145.4
(22) Date de dépôt: 01.09.2003
(51) Int. Cl.: C07C 227/32, C07C 229/16, C07D 265/32, C07C 229/12

(54) **Nouveau procédé de synthèse des esters de la N-((S)-1-carboxybutyl)-(S)-alanine et application à la synthèse du perindopril**
Neues Verfahren zur Herstellung von Estern des N-((S)-1-Carboxybutyl)-(S)-alanins und seine Verwendung in der Synthese von Perindopril
New process for the synthesis of N-((S)-1-carboxybutyl)-(S)-alanine esters and their use in the synthesis of perindopril

(43) Date de publication de la demande: 19.11.2003
(73) Titulaire: Les Laboratoires Servier, 92415 Courbevoie Cedex (FR)
(72) Inventeur: Breard, Fabienne, 76140 Petit Quevilly (FR); Fugier, Claude, 76210 Gruchet Le Valasse (FR)

(56) Documents cités:
- EP-A- 0 308 340
- BAKER, WILLIAM R. ET AL: "Synthesis of the nonpeptide renin inhibitor A-68064 and the ACE inhibitor methyl enalaprilat from (5S)-2,3,5,6-tetrahydro-5-alkyl-N-(tert- butyloxycarbonyl)-4H-1,4-oxazine-2-ones" TETRAHEDRON LETTERS (1992), 33(12), 1577-80 , XP002265185
- BAKER, WILLIAM R. ET AL: "Synthesis and structure determination of (3S,5S)-2,3,5,6-tetrahydro-3,5- dialkyl-N-(tert-butyloxycarbonyl)-4H-1,4-o xazine-2-ones" TETRAHEDRON LETTERS (1992), 33(12), 1573-6 , XP002265186

## Description

La présente invention concerne un procédé de synthèse des esters de la N-[(S)-1-carboxybutyl]-(S)-alanine, et leur application à la synthèse du perindopril et de ses sels pharmaceutiquement acceptables.

Plus spécifiquement, la présente invention concerne un nouveau procédé de synthèse des dérivés de formule (I) : 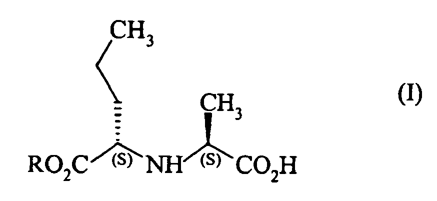 dans laquelle R représente un groupement alkyle (C₁-C₆) linéaire ou ramifié,
ainsi que leurs sels d'addition à un acide ou à une base minéral(e) ou organique.

Les composés de formule (I) obtenus selon le procédé de l'invention sont utiles dans la synthèse du perindopril de formule (II) : 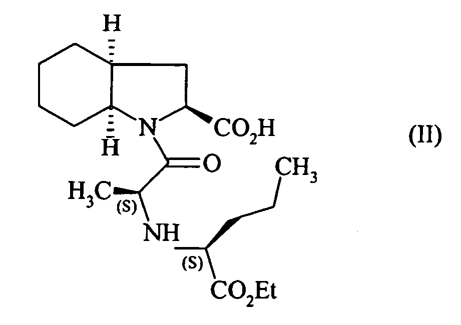 ainsi que dans celle de ses sels pharmaceutiquement acceptables.

Le perindopril, ainsi que ses sels, possèdent des propriétés pharmacologiques intéressantes. Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse performant, permettant notamment l'obtention sélective du diastéréoisomère (S,S) avec un bon rendement et une excellente pureté, mais également facilement transposable à l'échelle industrielle.

Quelques méthodes de préparation des composés de formule (I) sont déjà connues.
- Le journal Tet. Lett. 1982, 23 (16), 1677-80 décrit l'obtention d'un dérivé de formule (I) (R = éthyle) par réaction dans l'éthanol du 2-oxovalérate d'éthyle avec l'ester tert-buylique de l'alanine en présence de cyanoborohydrure de sodium.
- Le brevet EP 0 309 324 décrit l'accès à un composé de formule (I) (R = éthyle) par réaction dans le diméthylformamide de l'ester benzylique de l'alanine avec l'α-bromovalérate d'éthyle en présence de triéthylamine.
- Les brevets EP 0 308 340 et EP 0 308 341 décrivent l'accès à un composé de formule (I) (R = éthyle) par réaction dans l'eau du chlorhydrate de norvalinate d'éthyle avec l'acide pyruvique en présence d'hydrogène, de charbon palladié et de soude.

La demanderesse a présentement mis au point un nouveau procédé de synthèse industrielle des dérivés de formule (I).

Plus spécifiquement, la présente invention concerne un procédé de synthèse des composés de formule (I), caractérisé en ce que l'on met en réaction une morpholinone de formule (III) : 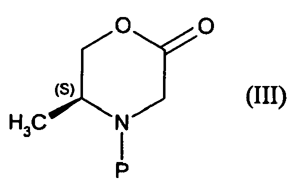 dans laquelle P représente un groupement protecteur de la fonction amino,
- soit avec le bromure ou le triflate d'allyle, en présence d'une base, pour conduire au composé de formule (IV), de configuration (3S, 5S) : 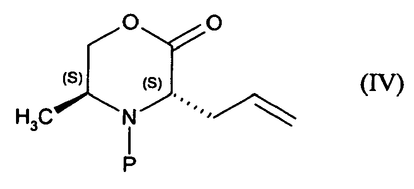
dans laquelle P est tel que défini précédemment,
que l'on hydrogène en présence de palladium sur charbon,
- soit avec l'iodopropane,
pour conduire au composé de formule (V), de configuration (3S, 5S) : 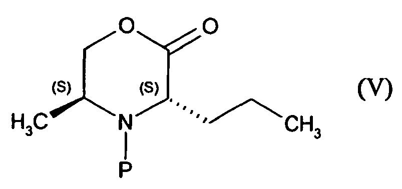 dans laquelle P est tel que défini précédemment,
que l'on soumet à l'action de LiOH, puis à l'action d'un réactif d'estérification,
pour conduire au composé de formule (VI) : 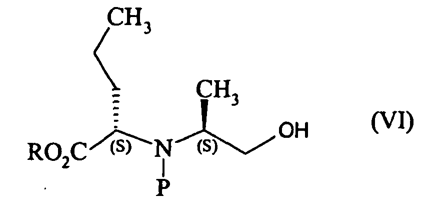 dans laquelle R et P sont tels que définis précédemment,
que l'on met en réaction avec un agent oxydant, pour conduire, après déprotection de la fonction amino, au composé de formule (I).

Parmi les groupements protecteurs de la fonction amino utilisables dans la présente invention, on peut citer à titre non limitatif les groupements tert-butyloxycarbonyle et benzyloxycarbonyle. Le groupement P préféré est le groupement tert-butyloxycarbonyle.

Parmi les bases utilisables pour la réaction entre le composé de formule (III) et le bromure ou le triflate d'allyle, on peut citer à titre non limitatif le diisopropylamidure de lithium (LDA), le bis(triméthylsilyl)amidure de sodium (NaHMDS) et le tert-butanolate de potassium.

Parmi les réactifs d'estérification utilisables pour la formation du composé de formule (VI), on peut citer à titre préférentiel les composés de formule (VII) :

R-X (VII)

dans laquelle R est tel que défini dans la formule (I), et X représente un groupement triflate, tosylate ou mésylate ou un atome d'halogène, préférentiellement l'iode.

Lorsqu'on souhaite accéder aux composés de formule (I) pour lesquels R représente un groupement méthyle, le réactif d'estérification peut également être le diazométhane.

Parmi les agents oxydants utilisables pour la réaction d'oxydation du composé de formule (VI), on peut citer à titre non limitatif NaIO₄ en présence de RuCl₃.

L'oxydation peut également être effectuée en deux étapes, en transformant dans un premier temps le composé de formule (VI) en aldéhyde correspondant, par exemple dans des conditions de Swern, puis en oxydant l'aldéhyde en acide carboxylique correspondant, par exemple par KMnO₄.

Les composés de formule (V) et (VI) sont des produits nouveaux, utiles comme intermédiaires de synthèse dans l'industrie chimique ou pharmaceutique, notamment dans la synthèse du perindopril, et font à ce titre partie intégrante de la présente invention.

Le groupement R préféré est le groupement éthyle.

Le composé de formule (III) peut être obtenu à partir de (S)-N-benzylalaninol, que l'on fait réagir avec le bromoacétate d'éthyle, en présence de triéthylamine, pour conduire, après clivage du groupement benzyle, au (S)-N-(éthoxycarbonylméthyl)alaninol, que l'on protège ensuite par le groupement P tel que défini précédemment, puis que l'on cyclise par réaction avec l'acide para-toluènesulfonique.

### Exemple : N-[(S)-Carbéthoxy-1-butyl]-(S)-alanine, chlorhydrate

### Stade A : (3S,5S)-3-Allyl-5-méthyl-2-oxo-4-morpholinecarboxylate de tert-butyle :

Dans un réacteur, charger 200 g de (5S)-5-méthyl-2-oxo-4-morpholinecarboxylate de tert-butyle et 700 ml de tétrahydrofurane, puis refroidir la solution à -60°C et ajouter 700 ml d'une solution 2M de diisopropylamidure de lithium dans le tétrahydrofurane et l'heptane, en maintenant la température du milieu réactionnel en-dessous de -40°C. Après 1 heure de réaction, ajouter 225 g de bromure d'allyle, tout en maintenant la température du mélange réactionnel à -30°C, et agiter pendant 3h.
Laisser ensuite remonter le mélange réactionnel à température ambiante, hydrolyser par une solution aqueuse de chlorure d'ammonium, extraire à l'éther et laver la phase éthérée par de l'eau.

Le (3S,5S)-3-allyl-5-méthyl-2-oxo-4-morpholinecarboxylate de tert-butyle isolé par mise à sec de la phase éthérée est utilisé tel quel dans l'étape suivante.

### Stade B : (3S,5S)-5-Méthyl-3-propyl-2-oxo-4-morpholinecarboxylate de tert-butyle :

Dans un hydrogénateur, placer 200 g du composé obtenu dans le stade précédent en solution dans l'éthanol, puis 5 g de Pd/C à 10 %. Hydrogéner sous pression normale et température ambiante, jusqu'à absorption de la quantité théorique d'hydrogène.
Eliminer le catalyseur par filtration, puis isoler le (3S,5S)-5-méthyl-3-propyl-2-oxo-4-morpholinecarboxylate de tert-butyle par mise à sec.

### Stade C : (2S)-2-{(Tert-butyloxycarbonyl)[(1S)-2-hydroxy-1-méthyléthyl]-amino}-pentanoate d 'éthyle

Dans un réacteur, charger 200 g du composé obtenu au stade précédent, 500 ml d'acétonitrile, 500 ml d'eau et 500 ml d'hexane, puis ajouter 33 g d'hydrate d'hydroxyde de lithium et agiter 3h à 0°C.
Le mélange réactionnel est alors mis à sec et le sel de lithium obtenu est mis en solution dans 1,5 1 de diméthylformamide, puis traité par 122 g d'iodoéthane à température ambiante.
Après évaporation de la diméthylformamide, le résidu de mise à sec est repris par l'éthanol et filtré sur silice, pour conduire au (2S)-2-{(tert-butyloxycarbonyl)[(1S)-2-hydroxy-1-méthyléthyl]-amino}-pentanoate d'éthyle avec un rendement de 60%.

### Stade D : N-[(S)-Carbéthoxy-1-butyl]-N-(tert-butyloxycarbonyl)-(S)-alanine

Dans un réacteur, charger 500 ml de dichlorométhane, 500 ml d'eau et 500 ml d'acétonitrile, puis ajouter 141 g de periodate de sodium et 1,35 g de trichlorure de ruthénium hydraté. Agiter 1h et ajouter rapidement 200 g du composé obtenu au stade précédent. En fin de réaction, filtrer sur Célite®, laver la phase organique et l'évaporer à sec, pour conduire à la N-[(S)-carbéthoxy-1-butyl]-N-(tert-butyloxycarbonyl)-(S)-alanine.

### Stade E : N-[(S)-Carbéthoxy-1-butyl]-(S)-alanine, chlorhydrate :

Dans un réacteur, placer 200 g du composé obtenu dans le stade précédent et 1,5 1 d'acétate d'éthyle, puis amener le milieu réactionnel à 0°C et faire passer un courant d'acide chlorhydrique gaz pendant 30 minutes. Après une nuit d'agitation à température ambiante, le précipité formé est filtré, rincé et séché, pour conduire au chlorhydrate de la N-[(S)-carbéthoxy-1-butyl]-(S)-alanine avec un rendement quantitatif.

## Revendications

1. Procédé de synthèse des composés de formule (I) 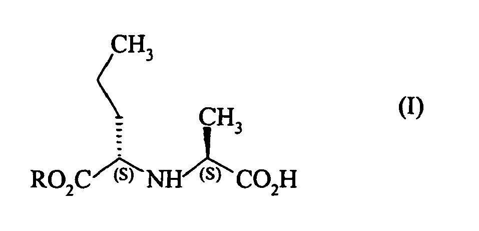 dans laquelle R représente un groupement alkyle (C₁-C₆) linéaire ou ramifié,
**caractérisé en ce que** l'on met en réaction une morpholinone de formule (III) : 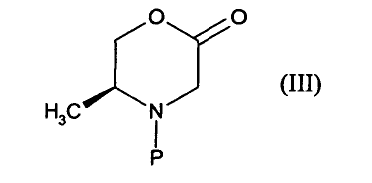 dans laquelle P représente un groupement protecteur de la fonction amino,
• soit avec le bromure ou le triflate d'allyle, en présence d'une base, pour conduire au composé de formule (IV), de configuration (3S, 5S) : 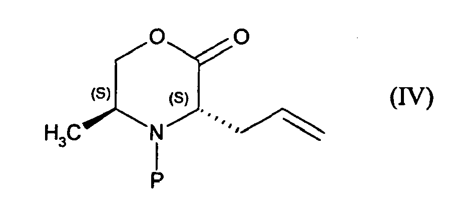
dans laquelle P est tel que défini précédemment,
que l'on hydrogène en présence de palladium sur charbon,
• soit avec l'iodopropane,
pour conduire au composé de formule (V) : 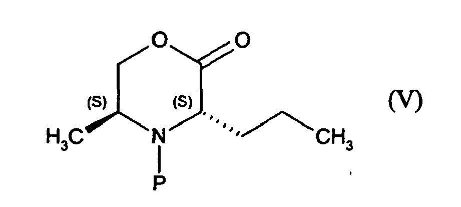
dans laquelle P est tel que défini précédemment,
que l'on soumet à l'action de LiOH, puis à l'action d'un réactif d'estérification,
pour conduire au composé de formule (VI) : 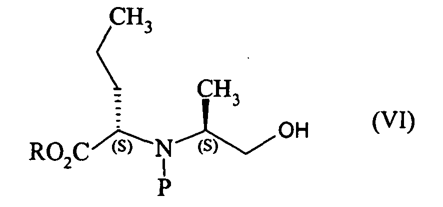 dans laquelle R et P sont tels que définis précédemment,
que l'on met en réaction avec un agent oxydant, pour conduire, après déprotection de la fonction amino, au composé de formule (I).

2. Procédé de synthèse selon la revendication 1, permettant l'obtention du dérivé de formule (I) dans laquelle R représente le groupement éthyle.

3. Procédé de synthèse selon l'une quelconque des revendications 1 ou 2, **caractérisé en ce que** P représente le groupement tert-butyloxycarbonyle.

4. Procédé de synthèse selon l'une quelconque des revendications 1 à 3, **caractérisé en ce que** la base utilisée pour la réaction entre le composé de formule (III) et le bromure ou le triflate d'allyle est le diisopropylamidure de lithium, le bis(triméthylsilyl)amidure de sodium ou le tert-butanolate de potassium.

5. Procédé de synthèse selon l'une quelconque des revendications 1 à 4, **caractérisé en ce que** le réactif d'estérification est l'iodoéthane.

6. Procédé de synthèse selon l'une quelconque des revendications 1 à 5, **caractérisé en ce que** l'agent oxydant est NaIO₄ en présence de RuCl₃.

7. Composé de formule (V) : 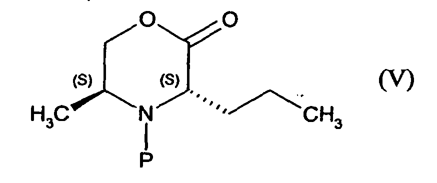 dans laquelle P représente le groupement tert-butyloxycarbonyle.

8. Composé de formule (VI) : 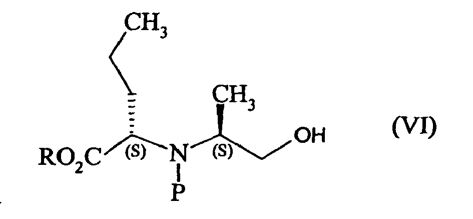 dans laquelle P représente le groupement tert-butyloxycarbonyle et R représente le groupement éthyle.

9. Procédé de synthèse du perindopril ou de ses sels pharmaceutiquement acceptables,
dans lequel le composé de formule (III) est transformé en intermédiaire de formule (I) selon le procédé de la revendication 1, puis ledit intermédiaire de formule (I) est transformé en perindopril ou en l'un de ses gels pharmaceutiquement acceptables.

## Claims

1. Process for the synthesis of the compounds of formula (I) 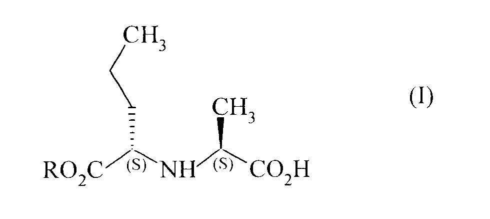 wherein R represents a linear or branched (C₁-C₆)alkyl group,
**characterised in that** a morpholinone of formula (III) : 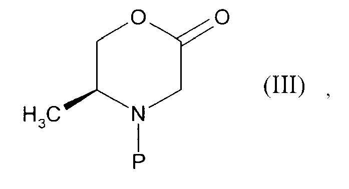 wherein P represents a protecting group for the amino function, is reacted
• either with allyl bromide or allyl triflate, in the presence of a base, to yield a compound of formula (IV) having the (3S,5S) configuration : 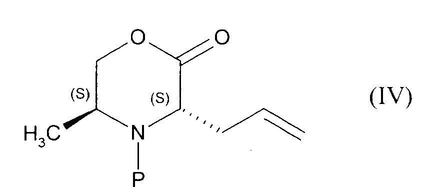
wherein P is as defined hereinbefore,
which is hydrogenated in the presence of palladium-on-carbon,
• or with iodopropane,
to yield a compound of formula (V) : 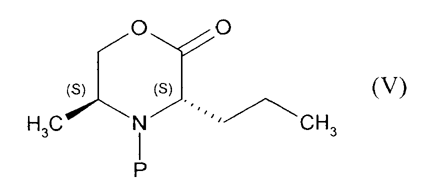
wherein P is as defined hereinbefore,
which is subjected to the action of LiOH, then to the action of an esterification reagent,
to yield a compound of formula (VI) : 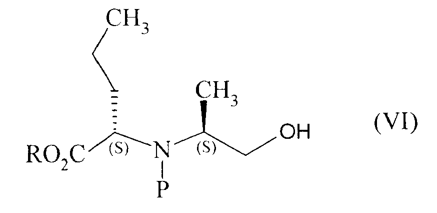 wherein R and P are as defined hereinbefore,
which is reacted with an oxidising agent to yield, after deprotection of the amino function, the compound of formula (I).

2. Synthesis process according to claim 1, allowing a compound of formula (I) wherein R represents an ethyl group to be obtained.

3. Synthesis process according to either claim 1 or claim 2, **characterised in that** P represents a tert-butoxycarbonyl group.

4. Synthesis process according to any one of claims 1 to 3, **characterised in that** the base used for the reaction between the compound of formula (III) and allyl bromide or allyl triflate is lithium diisopropylamide, sodium bis(trimethylsilyl)amide or potassium tert-butanolate.

5. Synthesis process according to any one of claims 1 to 4, **characterised in that** the esterification reagent is iodoethane.

6. Synthesis process according to any one of claims 1 to 5, **characterised in that** the oxidising agent is NaIO₄ in the presence of RuCl₃.

7. Compound of formula (V) : 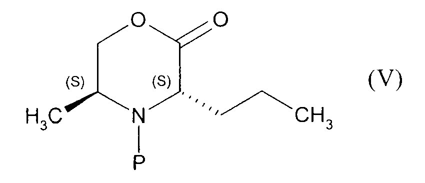 wherein P represents a tert-butoxycarbonyl group.

8. Compound of formula (VI) : 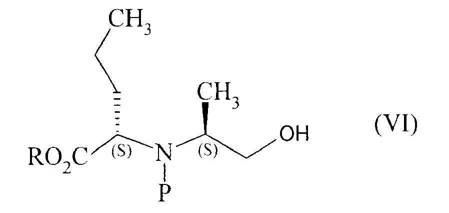 wherein P represents a tert-butoxycarbonyl group and R represents an ethyl group.

9. Process for the synthesis of perindopril or pharmaceutically acceptable salts thereof, wherein the compound of formula (III) is converted to an intermediate of formula (I) according to the process of claim 1, then the said intermediate of formula (I) is converted to perindopril or to one of its pharmaceutically acceptable salts.

## Patentansprüche

1. Verfahren zur Synthese der Verbindungen der Formel (I) 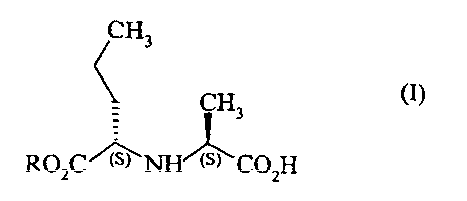 in der R eine geradkettige oder verzweigte (C₁-C₆)-Alkylgruppe darstellt,
**dadurch gekennzeichnet, dass** man ein Morpholinon der Formel (III): 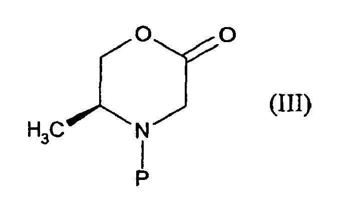 in der P eine Schutzgruppe der Aminofunktion darstellt,
• entweder mit Allylbromid oder Allyltriflat in Gegenwart einer Base umsetzt zur Bildung der Verbindung der Formel (IV) in der Konfiguration (3S, 5S): 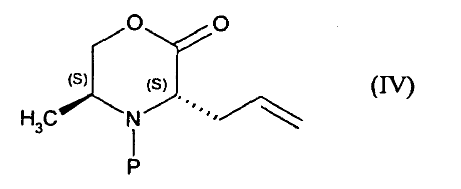 in der P die oben angegebene Bedeutung besitzt,
welche man in Gegenwart von Palladium-auf-Kohlenstoff hydriert,
• oder mit Iodpropan umsetzt
zur Bildung der Verbindung der Formel (V): 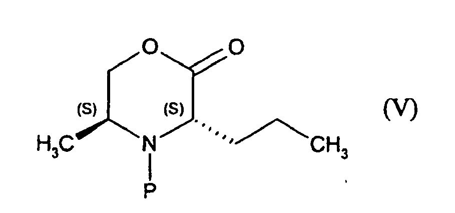 in der P die oben angegebene Bedeutung besitzt,
welche man der Einwirkung von LiOH und dann der Einwirkung eines Veresterungsreagens unterzieht zur Bildung der Verbindung der Formel (VI): 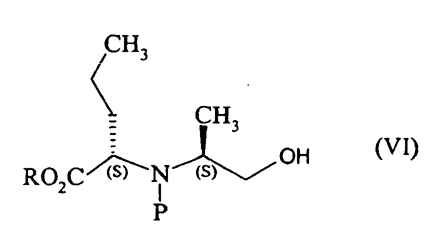 in der R und P die oben angegebenen Bedeutungen besitzen,
welche man der Einwirkung eines Oxidationsmittels unterwirft, sodass man nach der Abspaltung der Schutzgruppe der Aminofunktion die Verbindung der Formel (I) erhält.

2. Syntheseverfahren nach Anspruch 1 zur Bildung des Derivats der Formel (I), in der R die Ethylgruppe bedeutet.

3. Syntheseverfahren nach einem der Ansprüche 1 oder 2, **dadurch gekennzeichnet, dass** P die tert.-Butyloxycarbonylgruppe bedeutet.

4. Syntheseverfahren nach einem der Ansprüche 1 bis 3, **dadurch gekennzeichnet, dass** die für die Reaktion der Verbindung der Formel (III) mit Allylbromid oder Allyltriflat verwendete Base Lithiumdiisopropylamid, Natrium-bis(trimethylsilyl)-amid oder Kalium-tert.-butanolat ist.

5. Syntheseverfahren nach einem der Ansprüche 1 bis 4, **dadurch gekennzeichnet, dass** das Veresterungsreagens Iodethan ist.

6. Syntheseverfahren nach einem der Ansprüche 1 bis 5, **dadurch gekennzeichnet, dass** das Oxidationsmittel NaIO₄ in Gegenwart von RuCl₃ ist.

7. Verbindung der Formel (V): 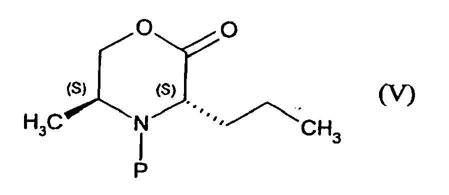 in der P die tert.-Butyloxycarbonylgruppe bedeutet.

8. Verbindung der Formel (VI): 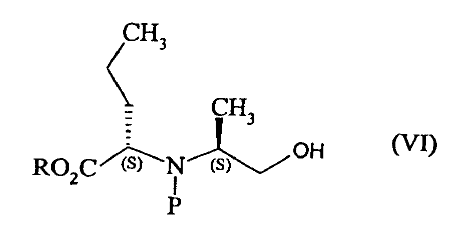 in der P die tert.-Butyloxycarbonylgruppe und R die Ethylgruppe bedeuten.

9. Verfahren zur Synthese von Perindopril oder seinen pharmazeutisch annehmbaren Salzen, bei dem die Verbindung der Formel (III) nach dem Verfahren des Anspruchs 1 in das Zwischenprodukt der Formel (I) umgewandelt wird und dann das Zwischenprodukt der Formel (I) in Perindopril oder eines seiner pharmazeutisch annehmbaren Salze umgewandelt wird.
